# EUROPEAN PATENT APPLICATION

(11) **EP 4 755 292 A1**
(43) Date of publication of application: **10.06.2026**
(21) Application number: 25213073.7
(22) Date of filing: 03.11.2025
(51) Int. Cl.: A61B 5/00, A61B 5/024, A61B 5/026, A61B 5/1455, H01S 5/068

(54) **CONSTANT CURRENT DRIVE FOR LASERS IN PHOTOPLETHYSMOGRAPHY**

(30) Priority: 05.12.2024 US 202418970587
(71) Applicant: Zynex Monitoring Solutions, Inc., Englewood, CO 80112 (US)
(72) Inventor: Pologe, Jonas Alexander, Boulder, CO 80305 (US)
(74) Representative: Forresters IP LLP

(57) **Abstract**

A photoplethysmographic monitor has two or more emitters wherein at least one of the emitters is a single longitudinal mode diode laser, electronic drive circuitry adapted to energize the at least one laser to a pre-determined constant current level, and a housing containing the at least one laser, the housing thermally coupled to a temperature-controlled element. The temperature-controlled element configured to hold the housing at a fixed temperature and the pre-determined constant current level of the at least one laser is selected to set the at least one laser, when energized and when held at the fixed temperature, to operate between longitudinal mode hop points of the at least one laser.

## Description

### FIELD OF THE INVENTION

This invention is in the field of photoplethysmography.

### BACKGROUND OF THE INVENTION

In the science of photoplethysmography, light is used to illuminate or trans-illuminate living tissue for the purpose of providing noninvasive measurements of blood analytes including but not limited to the levels of arterial oxyhemoglobin, carboxyhemoglobin, methemoglobin, reduced hemoglobin, and/or total hemoglobin. Additionally, photoplethysmography can be designed to measure various hemodynamic parameters, and/or tissue properties including, but not limited to, heart rate, respiratory rate, and perfusion.

In this monitoring modality multiple different spectral bands of light are directed into living tissue (the "tissue-under-test") and a portion of the light that is not absorbed by the tissue or scattered in some other direction is detected a short distance from the point at which the light entered the tissue. When light, at wavelengths that can be absorbed by hemoglobin or other components of arterial blood, passes through living tissue the light is modulated by the pulsatile arterial blood flow. The pulsatile (or photoplethysmographic) light signals exiting the tissue and picked up by the detector are converted into electronic signals (or photoplethysmographic signals or photoplethysmographic data) by a photodetector and are then used to calculate the desired blood analyte levels and/or hemodynamic parameters. A device which generates light to be emitted into the tissue and detects and processes the photoplethysmographic signals (or data) emitted by the tissue, to measure the levels of various blood analytes and/or various hemodynamic parameters, is a photoplethysmographic device. A photoplethysmographic device typically includes a photoplethysmographic monitor combined with a sensor. The first widely used commercial photoplethysmographic device was a pulse oximeter, a photoplethysmographic device designed to noninvasively measure, at least, arterial blood oxygen saturation. This device is now used in almost all areas of medicine.

The (photoplethysmographic) monitor includes electronic drive circuitry for controlling light emitters that emit light which is then incident on the tissue. The monitor also performs the functions of receiving and processing the photoplethysmographic signals emitted from the tissue, converting these photoplethysmographic data into the various blood analytes and/or hemodynamic parameter measurements, and displaying these measurements on some form of user display. Also included in any photoplethysmographic device is a sensor which is affixed to, or held in place against, the tissue to deliver light from the emitters to the tissue. The sensor further includes a photodetector for receiving the photoplethysmographic light signals from the tissue.

Typically, the sensor is connected to the monitor by a patient cable that has a connector to allow it to be removably connected to the monitor. Further, depending on the design of the patient cable, the sensor can be permanently connected to the distal end of patient cable, or the patient cable may have a connector on its distal end to connect and disconnect to the sensors.

Pulse oximeters most commonly use light emitting diodes (LEDs) for the emitters, to generate the light that is to be incident on the tissue. These diodes are typically positioned directly in the sensor within a few millimeters from the tissue of the person being monitored. The photodetector is also, most commonly, positioned directly in the sensor to maximize the received light levels. Some photoplethysmographic devices, however, position the photodetector and/or the emitters at a distance from the sensor and use light guides, such as fiber optics or one or more fiber optic bundles, to transmit the light from the emitters to the sensor and the photoplethysmographic signals, emitted by the tissue, from the sensor to the photodetector. This is particularly common in magnetic resonance imaging (MRI) compatible pulse oximetry sensors where it is helpful to keep the electronics away from the magnetic and electromagnetic fields generated and read by the MRI device.

These photoplethysmographic devices have worked well in almost all clinical settings and have made the pulse oximeter into a nearly universally used monitoring device in healthcare. However, using LEDs for photoplethysmographic measurement still comes with some limitations. The spectral content of LEDs, while centered at the required wavelengths, is very broad, typically about 100nm. These broadband light sources limit the measurement accuracy that can be attained by these LED-based pulse oximeters, cause pulse oximeters to read falsely elevated arterial oxygen saturations at low arterial oxygen saturation levels, and also limit the number of blood analytes that can be measured.

To make it possible to accurately measure multiple blood analytes, multiple different spectral bands of light are required, and the full power spectral bandwidth of (at least some of) these light sources, or emitters, should be as close to monochromatic as possible. Further the spectral content of these emitters must be fixed and stable.

These optical requirements can be met by replacing the LED light sources (or at least some of the LED light sources) for photoplethysmographic measurement with lasers, and, in particular, with one or more single longitudinal mode diode lasers, each of which typically have a spectral bandpass in the 1nm range, which are nearly monochromatic compared to LED emitters.

The use of lasers in photoplethysmography, however, has not been successfully achieved in the past. This is due, in part, because laser light tends to vary both in intensity and in spectral content making photoplethysmographic measurement almost impossible, given the degradation in the signal-to-noise ratio caused by these fluctuations. If lasers are to be used in a photoplethysmographic device, the light emitted by the lasers must be narrowband and highly stable, both in spectral content and in output intensity.

### SUMMARY OF THE INVENTION

The present invention overcomes the problems and disadvantages associated with current strategies and designs and provides new systems and methods of manufacturing photoplethysmographic devices.

One embodiment of the invention is directed to a photoplethysmographic monitor. The photoplethysmographic monitor includes two or more emitters wherein at least one of the emitters is a single longitudinal mode diode laser, electronic drive circuitry adapted to energize the at least one laser to a pre-determined constant current level, and a housing containing the at least one laser, the housing thermally coupled to a temperature-controlled element. The temperature-controlled element is configured to hold the housing at a fixed temperature and the pre-determined constant current level of the at least one laser is selected to set the at least one laser, when energized and when held at the fixed temperature, to operate between longitudinal mode hop points of the at least one laser.

In a preferred embodiment, the temperature-controlled element is configured to maintain the housing to within one degree centigrade of a predetermined temperature. Preferably, the electronic drive circuitry is further adapted to energize at least two of the two or more emitters by time-division-multiplexing. Preferably, the temperature-controlled element is a thermoelectric cooler or a resistive heater.

Another embodiment of the invention is directed to a method of assembling a photoplethysmographic monitor. The method includes the steps of providing a housing comprising at least one single longitudinal mode diode laser within the housing, the monitor further including at least one additional emitter, thermally coupling the housing to a temperature-controlled element, adapting a temperature controller to set the temperature-controlled element to hold the housing at a fixed temperature, coupling electronic drive circuitry to the at least one laser, the electronic drive circuitry adapted to energize the at least one laser to a pre-determined constant current level, setting the pre-determined constant current level of the at least one laser to a level between longitudinal mode hop points when energized and when held at the fixed temperature.

In a preferred embodiment, the temperature-controlled element is configured to maintain the housing to within one degree centigrade of a predetermined temperature. Preferably, the electronic drive circuitry is further adapted to energize at least two of the two or more emitters by time-division-multiplexing. Preferably, the temperature-controlled element is a thermoelectric cooler or a resistive heater.

Another embodiment of the invention is directed to a photoplethysmographic monitor. The photoplethysmographic monitor includes two or more emitters wherein at least one of the emitters is a single longitudinal mode diode laser, electronic drive circuitry adapted to energize the at least one laser to a pre-determined constant current level, and a housing containing the at least one laser, the housing thermally coupled to a temperature-controlled element. The temperature-controlled element is configured to hold the housing at a fixed temperature such that, when the at least one laser is energized to the pre-determined constant current level, the at least one laser operates between longitudinal mode hop points.

In a preferred embodiment, the temperature-controlled element is configured to maintain the housing to within one degree centigrade of a predetermined temperature. Preferably, the electronic drive circuitry is further adapted to energize at least two of the two or more emitters by time-division-multiplexing. Preferably, the temperature-controlled element is a thermoelectric cooler or a resistive heater.

Another embodiment of the invention is directed to a method of assembling a photoplethysmographic monitor. The method includes the steps of providing a housing comprising at least one single longitudinal mode diode laser within the housing, the monitor further including at least one additional emitter, coupling electronic drive circuitry to the at least one laser, the electronic drive circuitry adapted to energize the at least one laser to a pre-determined constant current level, thermally coupling the housing to a temperature-controlled element, and adapting a temperature controller to set the temperature-controlled element to hold the housing at a fixed temperature such that, when the at least one laser is energized to the pre-determined constant current level, the at least one laser operates between longitudinal mode hop points.

In a preferred embodiment, the temperature-controlled element is configured to maintain the housing to within one degree centigrade of a predetermined temperature. Preferably, the electronic drive circuitry is further adapted to energize at least two of the two or more emitters by time-division-multiplexing. Preferably, the temperature-controlled element is a thermoelectric cooler or a resistive heater.

Other embodiments and advantages of the invention are set forth in part in the description, which follows, and in part, may be obvious from this description, or may be learned from the practice of the invention.

### REPRESENTATIVE FEATURES

Representative features are set out in the following clauses, which stand alone or may be combined, in any combination, with one or more features disclosed in the text and/or drawings of the specification.
1. A photoplethysmographic monitor, comprising:
   two or more emitters wherein at least one of the emitters is a single longitudinal mode diode laser;
   electronic drive circuitry adapted to energize the at least one laser to a pre-determined constant current level; and
   a housing containing the at least one laser, the housing thermally coupled to a temperature-controlled element;
   the temperature-controlled element configured to hold the housing at a fixed temperature; the pre-determined constant current level of the at least one laser is selected to set the at least one laser, when energized and when held at the fixed temperature, to operate between longitudinal mode hop points of the at least one laser.
2. The monitor of clause 1, wherein the temperature-controlled element is configured to maintain the housing to within one degree centigrade of a predetermined temperature.
3. The monitor of clause 1 or 2, wherein the electronic drive circuitry is further adapted to energize at least two of the two or more emitters by time-division-multiplexing.
4. The monitor of clause 1, 2, or 3, wherein the temperature-controlled element is a thermoelectric cooler.
5. The monitor of clause 1, 2, or 3, wherein the temperature-controlled element is a resistive heater.
6. A method of assembling a photoplethysmographic monitor, comprising:
   providing a housing comprising at least one single longitudinal mode diode laser within the housing, the monitor further including at least one additional emitter;
   thermally coupling the housing to a temperature-controlled element;
   adapting a temperature controller to set the temperature-controlled element to hold the housing at a fixed temperature;
   coupling electronic drive circuitry to the at least one laser, the electronic drive circuitry adapted to energize the at least one laser to a pre-determined constant current level; and
   setting the pre-determined constant current level of the at least one laser to a level between longitudinal mode hop points when energized and when held at the fixed temperature.
7. The method of clause 6, wherein the temperature-controlled element is configured to maintain the housing to within one degree centigrade of a predetermined temperature.
8. The method of clause 6 or 7, wherein the electronic drive circuitry is further adapted to energize at least two of the two or more emitters by time-division-multiplexing.
9. The method of clause 6, 7, or 8, wherein the temperature-controlled element is a thermoelectric cooler.
10. The method of clause 6, 7, or 8, wherein the temperature-controlled element is a resistive heater.
11. A photoplethysmographic monitor, comprising:
   two or more emitters wherein at least one of the emitters is a single longitudinal mode diode laser;
   electronic drive circuitry adapted to energize the at least one laser to a pre-determined constant current level; and
   a housing containing the at least one laser, the housing thermally coupled to a temperature-controlled element;
   the temperature-controlled element configured to hold the housing at a fixed temperature such that, when the at least one laser is energized to the pre-determined constant current level, the at least one laser operates between longitudinal mode hop points.
12. The monitor of clause 11, wherein the temperature-controlled element is configured to maintain the housing to within one degree centigrade of a predetermined temperature.
13. The monitor of clause 11 or 12, wherein the electronic drive circuitry is further adapted to energize at least two of the two or more emitters by time-division-multiplexing.
14. The monitor of clause 11, 12, or 13, wherein the temperature-controlled element is a thermoelectric cooler.
15. The monitor of clause 11, 12, or 13, wherein the temperature-controlled element is a resistive heater.
16. A method of assembling a photoplethysmographic monitor, comprising:
   providing a housing comprising at least one single longitudinal mode diode laser within the housing, the monitor further including at least one additional emitter;
   coupling electronic drive circuitry to the at least one laser, the electronic drive circuitry adapted to energize the at least one laser to a pre-determined constant current level;
   thermally coupling the housing to a temperature-controlled element; and
   adapting a temperature controller to set the temperature-controlled element to hold the housing at a fixed temperature such that, when the at least one laser is energized to the pre-determined constant current level, the at least one laser operates between longitudinal mode hop points.
17. The method of clause 16, wherein the temperature-controlled element is configured to maintain the housing to within one degree centigrade of a predetermined temperature.
18. The method of clause 16 or 17, wherein the electronic drive circuitry is further adapted to energize at least two of the two or more emitters by time-division-multiplexing.
19. The method of clause 16, 17, or 18, wherein the temperature-controlled element is a thermoelectric cooler.
20. The method of clause 16, 17, or 18, wherein the temperature-controlled element is a resistive heater.

### BRIEF DESCRIPTION OF THE DRAWINGS

The foregoing features of embodiments will be more readily understood by reference to the following detailed description, taken with reference to the accompanying drawings, which show:
*FIG. 1**:* Photoplethysmographic device including monitor, patient cable, and sensor.
*FIG.* 2: Mode hop diagram for a single longitudinal mode diode laser.

### DETAILED DESCRIPTION OF THE INVENTION

As embodied and broadly described herein, the disclosures herein provide detailed embodiments of the invention. However, the disclosed embodiments are merely exemplary of the invention that may be embodied in various and alternative forms. Therefore, there is no intent that specific structural and functional details should be limiting, but rather the intention is that they provide a basis for the claims and a representative basis for teaching one skilled in the art to variously employ the present invention.

There is provided a photoplethysmographic monitor has two or more emitters wherein at least one of the emitters is a single longitudinal mode diode laser, electronic drive circuitry adapted to energize the at least one laser to a pre-determined constant current level, and a housing containing the at least one laser, the housing thermally coupled to a temperature-controlled element. The temperature-controlled element configured to hold the housing at a fixed temperature and the pre-determined constant current level of the at least one laser is selected to set the at least one laser, when energized and when held at the fixed temperature, to operate between longitudinal mode hop points of the at least one laser.

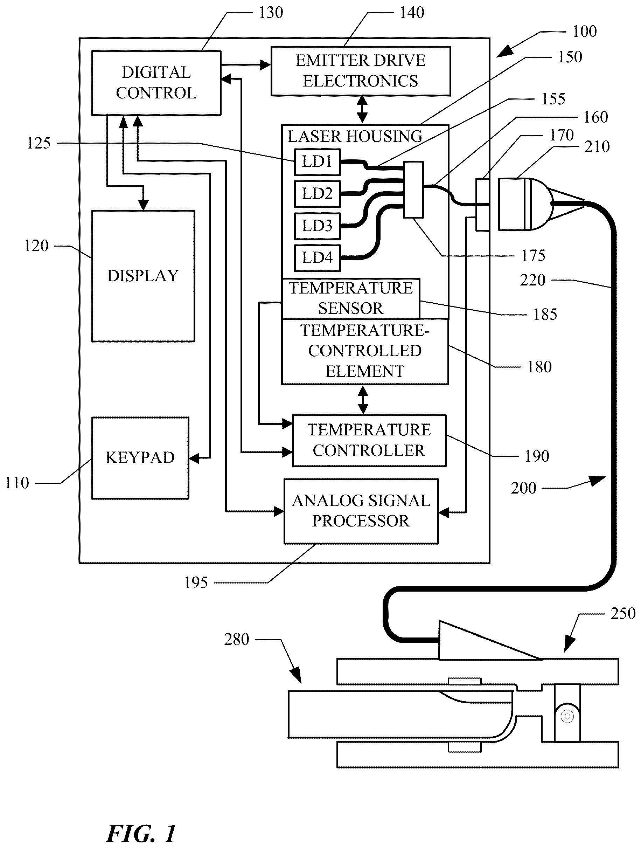

Photoplethysmographic devices, such as that shown schematically in Fig. 1, come in many forms including, but not limited to: a standalone monitor **100** having a built-in display **120** and detachable sensor **250,** or a photoplethysmographic device that transmits its measurements for display to, a remote display, an attached computer, or, wirelessly, to another display device such as a smart phone.

The photoplethysmographic monitor **100** may also provide other functions and include other components such as a keypad **110,** buttons, or touchscreen for user input, visible indicators (e.g. LED lights), audible enunciators (e.g. speakers) for alarms, and/or wired or wireless connection ports (e.g. USB, RS232, Ethernet, Bluetooth) for digital and analog inputs and outputs.

The photoplethysmographic device shown in FIG. 1 includes a photoplethysmographic monitor **100,** with display **120** and various controls **110** for operating monitor **100,** patient cable **200,** and sensor **250.** Monitor connector **170,** is designed to connect, or mate, to patient cable connector **210** to pass signals from the monitor **100** to sensor **250** and to pass signals from sensor **250** to monitor **100.** Patient cable **200** in Fig.1 includes patient cable connector **210,** cable **220,** and sensor **250.** In some embodiments, an additional connector may exist between cable **220** and sensor **250,** to allow cable **220** to remain connected to monitor **100** while sensor **250** is swapped out for an alternate sensor. In some embodiments, patient cable **200** may be hardwired or fixed to monitor **100** or patient cable **200** and sensor **250** may be integral to monitor **100** as is commonly seen in fingertip pulse oximeters where the sensor, monitor, display, and controls all reside in the same small housing, typically designed as a finger clip for clipping onto a person's finger for patient monitoring.

The photoplethysmographic device shown in FIG. 1 includes at least one single longitudinal mode diode laser **125,** although in FIG. 1 four such lasers **125** are shown as LD1 through LD4. This narrow spectral band laser(s) **125** generates light, that is transmitted to sensor **250,** to probe a tissue-under-test **280** in the sensor. Tissue **280** is meant to represent a finger, or other tissue such as an earlobe, cheek, forehead, or toe, positioned in, or against, sensor **250.** The laser, or lasers, **125** are typically positioned at a distance from sensor **250.** Thus, light from the laser(s) would be transmitted by light guide(s) **155,** which preferably is an optical fiber(s). Light guide(s) **155** transmits light from laser(s) **125** to monitor connector **170** where, when patient cable **200** is connected to monitor **100,** the light is coupled to one or more light guides within cable **220.** Cable **220** transmits the light from the laser(s) **125** to sensor **250** and, when the sensor is positioned on a patient, into tissue **280.**

Single longitudinal mode diode lasers may also be referred to as laser diodes or semiconductor lasers, but in this invention we are referring to very narrow band lasers that preferably have a single dominant output light wavelength.

Note that a single light guide **160** may be used to couple the light from multiple lasers **125** to the sensor **250.** For example, as shown schematically in FIG. 1, there could be four light guides **155** conducting light from each individual laser **125** coupled into a single light guide **160** for transmitting light from lasers **125** to sensor **250.** In this example, laser housing **150** includes a four-to-one optical coupler **175,** allowing the four light guides **155,** one from each of the four lasers **125,** to be coupled into a single light guide **160.** In other embodiments, the coupler **175** could be positioned at, or be created by, the front panel connector **170** to patient cable connector **210** interface. Alternatively, coupler **175** could be somewhere else along the optical path from lasers **125** to sensor **250.** Finally, individual light guides could be used for one or more of lasers **125** to transmit light from lasers **125** to sensor **250.**

In some embodiments, the laser(s) **125** are housed within monitor **100,** but in alternate embodiments the lasers could be located in other places, for example in a housing some distance from monitor **100** and connected to monitor **100** by a cable or by a wireless connection.

Regardless of the exact location of the laser(s) **125** in the photoplethysmographic device it may be desirable to be able to separate the connection between the sensor and the laser(s). In the embodiment shown in Fig. 1, the laser(s) **125** resides in monitor **100** and the separable connection is between monitor connector **170** and patient cable connector **210.** This allows for different types of sensors to be used on different patients or on different locations on a given patient. For example, an ear sensor may be more appropriate on one patient and a finger sensor on another.

One of the concerns in the design of any photoplethysmographic device is the stability of the light used to probe the tissue. The light must be stable in both intensity and spectral content. In the embodiments described herein, at least one of the emitters used to generate the light used by this photoplethysmographic device to probe tissue **280** is a single longitudinal mode laser **125.** These types of lasers can exhibit a behavior called "mode hopping" wherein there are rapid fluctuations in both intensity and spectral content. This mode hopping can be exacerbated by external effects such as optical feedback into the laser cavity or temperature changes of the laser.

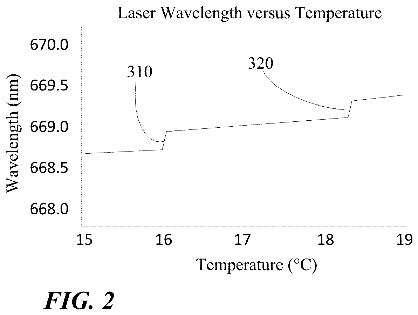

FIG. 2 is a characteristic plot of wavelength versus temperature for a single longitudinal mode laser showing two mode hop points **310** and **320.** The exact position, in temperature, and amplitude of these mode hops, and the difference in temperature between these mode hop points, varies with the laser type, laser construction, and even between two lasers made from the same semiconductor wafer and assembled under the same manufacturing processes.

In this embodiment, to ensure stability of the light sources in both amplitude and spectral content, the lasers are driven at a constant current and held at a fixed temperature, both of which are preferably set to hold laser **125** to an operating point between mode hop points. Temperature control is accomplished by locating the laser (or lasers) in a laser housing **150** and controlling the temperature of laser housing **150** with a temperature-controlled element **180.** Temperature-controlled element **180** is preferably a thermoelectric cooler thermally coupled to laser housing **150.** The thermal coupling can be accomplished by placing temperature-controlled element **180** in direct physical contact with laser housing **150.** This physical contact can be accomplished through adhesive bonding (with a thermally conductive epoxy or other adhesive that allows good thermal conductivity between these two elements), soldering, or clamping cooler **180** and the housing **150** together. A temperature controller **190** then controls the temperature-controlled element **180** to hold the laser housing at a predetermined fixed temperature. There is also, preferably, a temperature sensor **185,** such as a thermistor, embedded in laser housing **150** feeding the temperature of the housing back to temperature controller **190** to allow precise control of the temperature-controlled element to hold laser housing **150** at a fixed temperature. Note that while the embodiment shown in FIG. 1 employs a single laser housing **150** and a single temperature-controlled element **180,** and a single temperature sensor **185,** alternate configurations can be used which have multiple laser housings **150** each with its own temperature sensor **185,** and one or more temperature controllers **180,** so that different lasers **125** can be held at different fixed temperatures. Regardless of how many laser housings are employed, the housings can be held at a fixed temperature to within ± 1.0°C, ±0.5°C, ± 0.25°C or even ±0.1°C by the temperature controller **190** and the temperature-controlled element(s) **180.**

Holding lasers **125** at a temperature cooler than room temperature is preferable as it extends the lifetime of the lasers **125.** It is possible, however, to select a fixed temperature greater than room temperature and to hold the lasers at this temperature through the use of a resistive heater as the temperature-controlled element **180.** The advantage of this configuration is that it is every inexpensive and requires very simple electronics to control although the tradeoff may be shorter laser lifetimes and reduced output optical power.

Holding laser housing **150** at a fixed temperature, however, is not sufficient. Because the junction temperature of laser(s) **125** is also highly dependent on the drive current of the laser(s), it is necessary to energize, or drive, laser(s) **125** at a fixed, predetermined, constant drive current. It is the combination of the fixed temperature and the predetermined constant drive current(s) that ensures that laser(s) **125** will operate at a stable point between longitudinal mode hops.

While both the temperature and the drive current need to be fixed, adjusting either one will move the operating point of the laser **125.** Therefore, both the temperature setting and the drive current are predetermined to ensure an optimal operating point for laser **125.** For optimal performance of the laser the drive current is preferably set to a predetermined level that creates the desired power and single longitudinal mode output from laser **125,** and then the exact operating temperature is fine tuned to ensure that the laser is being operated away from mode hop points.

It is also possible to set the operating temperature of laser housing **150** first and then determine what the predetermined constant drive current(s) should be to get the desired output power and single longitudinal mode output from laser(s) **125** while simultaneously ensuring that laser(s) **125** is operating away from mode hop points.

The emitter drive electronics **140** drives the emitters in the photoplethysmographic device at the predetermined constant drive current levels. Preferably, the drive current for each emitter in the photoplethysmographic device can have its own predetermined constant drive current. The emitters may include not only lasers but also LEDs. The drive current for the emitters may be time division multiplexed wherein each emitter is driven at its predetermined drive current level and then that emitter is turned off and the next emitter is turned on sequentially with all emitters cycled through being turned on and off many times per second.

Control of all systems in the photoplethysmographic device is handled by the digital control **130** section. As indicated by the arrows in FIG. 1, digital control **130** talks to, receives data from, and/or controls at least display **120,** keypad **110,** emitter drive electronics **140,** temperature controller **190,** and analog signal processor **195.**

The analog signal processor **195** is preferably responsible for receiving and processing the photoplethysmographic light signals received from the tissue and converted into an electronic photoplethysmographic signals by the photodetector in the sensor. Then the analog signal processor **195** in combination with the digital control **130** can convert these photoplethysmographic data into blood analyte levels to be output to a clinician via display **120** or other display or output means.

Other embodiments and uses of the invention will be apparent to those skilled in the art from consideration of the specification and practice of the invention disclosed herein. All references cited herein, including all publications, U.S. and foreign patents and patent applications, are specifically and entirely incorporated by reference. It is intended that the specification and examples be considered exemplary only with the true scope of the invention indicated by the following claims. Furthermore, the term "comprising of" includes the terms "consisting of" and "consisting essentially of."

When used in this specification and claims, the terms "comprises" and "comprising" and variations thereof mean that the specified features, steps or integers are included. The terms are not to be interpreted to exclude the presence of other features, steps or components.

The invention may also broadly consist in the parts, elements, steps, examples and/or features referred to or indicated in the specification individually or collectively in any and all combinations of two or more said parts, elements, steps, examples and/or features. In particular, one or more features in any of the embodiments described herein may be combined with one or more features from any other embodiment(s) described herein.

Protection may be sought for any features disclosed in any one or more published documents referenced herein in combination with the present disclosure.

Although certain example embodiments of the invention have been described, the scope of the appended claims is not intended to be limited solely to these embodiments. The claims are to be construed literally, purposively, and/or to encompass equivalents.

## Claims

1. A photoplethysmographic monitor, comprising:
two or more emitters wherein at least one of the emitters is a single longitudinal mode diode laser;
electronic drive circuitry adapted to energize the at least one laser to a pre-determined constant current level; and
a housing containing the at least one laser, the housing thermally coupled to a temperature-controlled element;
the temperature-controlled element configured to hold the housing at a fixed temperature;
the pre-determined constant current level of the at least one laser is selected to set the at least one laser, when energized and when held at the fixed temperature, to operate between longitudinal mode hop points of the at least one laser.

2. The monitor of claim 1, wherein the temperature-controlled element is configured to maintain the housing to within one degree centigrade of a predetermined temperature.

3. The monitor of claim 1 or 2, wherein the electronic drive circuitry is further adapted to energize at least two of the two or more emitters by time-division-multiplexing.

4. The monitor of claim 1, 2, or 3, wherein the temperature-controlled element is a thermoelectric cooler.

5. The monitor of claim 1, 2, or 3, wherein the temperature-controlled element is a resistive heater.

6. A method of assembling a photoplethysmographic monitor, comprising:
providing a housing comprising at least one single longitudinal mode diode laser within the housing, the monitor further including at least one additional emitter;
thermally coupling the housing to a temperature-controlled element;
adapting a temperature controller to set the temperature-controlled element to hold the housing at a fixed temperature;
coupling electronic drive circuitry to the at least one laser, the electronic drive circuitry adapted to energize the at least one laser to a pre-determined constant current level; and
setting the pre-determined constant current level of the at least one laser to a level between longitudinal mode hop points when energized and when held at the fixed temperature.

7. The method of claim 6, wherein the temperature-controlled element is configured to maintain the housing to within one degree centigrade of a predetermined temperature.

8. The method of claim 6 or 7, wherein the electronic drive circuitry is further adapted to energize at least two of the two or more emitters by time-division-multiplexing.

9. The method of claim 6, 7, or 8, wherein the temperature-controlled element is a thermoelectric cooler or a resistive heater.

10. A photoplethysmographic monitor, comprising:
two or more emitters wherein at least one of the emitters is a single longitudinal mode diode laser;
electronic drive circuitry adapted to energize the at least one laser to a pre-determined constant current level; and
a housing containing the at least one laser, the housing thermally coupled to a temperature-controlled element;
the temperature-controlled element configured to hold the housing at a fixed temperature such that, when the at least one laser is energized to the pre-determined constant current level, the at least one laser operates between longitudinal mode hop points.

11. The monitor of claim 10, wherein the temperature-controlled element is configured to maintain the housing to within one degree centigrade of a predetermined temperature.

12. The monitor of claim 10 or 11, wherein the electronic drive circuitry is further adapted to energize at least two of the two or more emitters by time-division-multiplexing.

13. The monitor of claim 10, 11, or 12, wherein the temperature-controlled element is a thermoelectric cooler or a resistive heater.

14. A method of assembling a photoplethysmographic monitor, comprising:
providing a housing comprising at least one single longitudinal mode diode laser within the housing, the monitor further including at least one additional emitter;
coupling electronic drive circuitry to the at least one laser, the electronic drive circuitry adapted to energize the at least one laser to a pre-determined constant current level;
thermally coupling the housing to a temperature-controlled element; and
adapting a temperature controller to set the temperature-controlled element to hold the housing at a fixed temperature such that, when the at least one laser is energized to the pre-determined constant current level, the at least one laser operates between longitudinal mode hop points.

15. The method of claim 14, wherein the temperature-controlled element is configured to maintain the housing to within one degree centigrade of a predetermined temperature; and/or wherein the electronic drive circuitry is further adapted to energize at least two of the two or more emitters by time-division-multiplexing; and/or
wherein the temperature-controlled element is a thermoelectric cooler or a resistive heater.
